(19) Europäisches Patentamt
European Patent Office
Office européen des brevets

(11) **EP 4 224 487 A1**

(12) **EUROPEAN PATENT APPLICATION**

(43) Date of publication:
**09.08.2023  Bulletin 2023/32**

(21) Application number: **22155540.2**

(22) Date of filing: **08.02.2022**

(51) International Patent Classification (IPC):
**G16H 30/40** $^{(2018.01)}$  **G06T 17/00** $^{(2006.01)}$
**G16H 50/50** $^{(2018.01)}$

(52) Cooperative Patent Classification (CPC):
**G16H 50/50; A61B 5/367; G06T 17/00;**
**G16H 30/40;** A61B 5/055; A61B 6/03; A61B 6/032;
A61B 6/503; G06T 2210/41

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Karlsruher Institut für Technologie**
**76131 Karlsruhe (DE)**

(72) Inventors:
• **Azzolin, Luca**
  **76137 Karlsruhe (DE)**
• **Loewe, Axel**
  **76149 Karlsruhe (DE)**

• **Doessel, Olaf**
  **76646 Bruchsal (DE)**
• **Nairn, Deborah**
  **76131 Karlsruhe (DE)**
• **Nagel, Claudia**
  **76137 Karlsruhe (DE)**
• **Sánchez Arciniegas, Jorge Patricio**
  **76131 Karlsruhe (DE)**
• **Unger, Laura**
  **75045 Walzbachtal (DE)**
• **Zheng, Tianbao**
  **76131 Karlsruhe (DE)**

(74) Representative: **Gille Hrabal**
**Partnerschaftsgesellschaft mbB**
**Patentanwälte**
**Brucknerstraße 20**
**40593 Düsseldorf (DE)**

(54) **METHOD FOR GENERATING AN ANATOMICALLY AND FUNCTIONALLY PERSONALIZED COMPUTATIONAL ATRIA MODEL**

(57)    The invention relates to a method for generating an anatomically and functionally personalized computational model of a subject's heart (atria), i.e. a cardiac digital twin or virtual replica, to data processing devices and computer programs for carrying out the method and its use for carrying out in-silico experiments on the subject's cardiac digital twin, such as e.g. evaluation or prediction of cardiovascular treatment options or personalized therapy options.

FIG. 1

EP 4 224 487 A1

**Description**

**INTRODUCTION**

The invention relates to a method for generating an anatomically and functionally personalized computational model of a subject's heart (atria), i.e. a cardiac digital twin or virtual replica, to data processing devices and computer programs for carrying out the method, medical devices and computer programs leveraging cardiac digital twins, and its use for carrying out in-silico experiments on the subject's cardiac digital twin, such as e.g. evaluation or prediction of cardiovascular treatment options or personalized therapy options.

**BACKGROUND OF THE INVENTION**

Digital twins of a subject's heart are helpful tools for carrying out in-silico experiments on the subject's heart, for example for assessing arrhythmia vulnerability, for evaluating and predicting cardiovascular treatment options, and for developing personalized therapy options, in particular for cardiac arrhythmia treatment and related risk assessment. Atrial fibrillation (AF) is a cardiac arrhythmia characterized by uncoordinated and chaotic atrial activation affecting more than 40 million people worldwide. AF is the most prevalent arrhythmia and is associated with an increased long-term risk of other cardiovascular diseases. Computational modelling provides a novel framework to assess initiation, maintenance, and progression of AF in a personalized manner. By computational modelling it becomes possible to create a personalized computational model, also called (cardiac) digital twin or virtual replica, of a subject's heart (atria). Such cardiac digital twins (CDT) are digital replicas of a subject's heart systematically integrating clinical data that match like-for-like all available clinical observations. Due to their intrinsic predictive ability, CDTs are a promising tool for precision medicine and personalised treatment aiding clinical decision making and providing an efficient and cost-effective platform for testing ethically and safely innovative therapies.

However, the process of building personalized computational models can be challenging and, so far, requires a high level of human interaction. It is therefore desirable to obtain a standardized and highly automated method for the generation of a subject's atrial digital twin from clinical data.

**PRIOR ART**

Current frameworks to deliver CDT integrating both the anatomical and functional twinning phases, referring to the inference of model anatomy and electrophysiology from clinical data, are not sufficiently efficient, robust, and accurate for advanced clinical and industrial applications. Several techniques to generate different sorts of atrial models have been presented covering a range of complexity and level of detail. Personalized computational models have been developed from either imaging data, e.g. derived from computed tomography (CT) or magnetic resonance images (MRI), or electroanatomical maps. But building such models directly from clinical geometrical data remains a challenging process since imaging data can be influenced by various degrees of segmentation uncertainty and electroanatomical maps can miss relevant anatomical structures.

The so-called Cemrg application "CemrgApp" (https://www.cemrg.co.uk/index.html) is an open source platform for image processing, which allows to provide MRI segmentation including fibrotic tissue distribution derived from late gadolinium enhancement (LGE) intensity in a semiautomatic and user-friendly way. However, CemrgApp accepts only DICOM® images, a specific standard format for transmitting, storing, retrieving, printing, processing and displaying medical imaging information, and requires a high degree of manual selection steps.

The so-called OpenEP application (https://openep.io/) is an open source platform to import, preprocess and analyze electroanatomical mapping data. However, OpenEP accepts only electroanatomical data for building the computational model and also requires a high degree of manual selection steps.

The required high degree of manual selection steps in both, CemregApp and OpenEP, limits the automatization of the model generation process significantly and makes it susceptible to user errors and user-dependent deviations in the outcome.

Corrado et al., "A work flow to build and validate patient specific left atrium electrophysiology models from catheter measurements", Medical Image Analysis, 2018 (doi: https://doi.org/10.1016/j.media. 2018.04.005) describe a method for building a patient's specific left atrium model using electroanatomical data, concretely catheter derived (invasive) mapping data.

Pheiffer et al. "Estimation of Local Conduction Velocity from Myocardium Activation Time: Application to Cardiac Resynchronization Therapy", International Conference on Functional Imaging and Modelling of the Heart, 2017 (doi: 239-248. 10.1007/978-3-319-59448-4_23) applied a back-propagation method on ventricular local activation time maps mapped onto a 2D disk. The disadvantage of using a back-propagation is the over-fitting of the model and the wave propagation is approximated with the shortest path.

Boyle et al. "Computationally guided personalized targeted ablation of persistent atrial fibrillation", Nat. Biomed. Eng., 2019 (doi: 10.1038/s41551-019-0437-9) describe a method to generate an atrial computational model based on information coming from LGE-MRI only. However, recent studies confirmed the low correlation between fibrosis distribution and LGE intensity in the atria (Stegmann et al., Europace, 2018). A fixed conduction velocity (CV) value of 0.43 m/s is given to the non-fibrosis areas. No further personalization of the conduction velocity is performed. Therewith, the model described by Boyle et al. allows only a low degree of personalization.

So, therewith, a standardized, automated process for generating personalised atrial computer models from geometries derived from different, preferably non-invasive, recording modalities remains unavailable.

Nagel et al., "A bi-atrial statistical shape model for large-scale in silico studies of human atria: model development and application to ECG simulations", Medical Image Analysis, 2021 (doi: 10.1016/j.media.2021.102210) describe a bi-atrial statistical shape model (SSM) covering the relevant anatomical variability required for in-silico electrophysiological experiments and well generalizing to unseen geometries. Moreover, SSM were shown to be a valuable tool to generate large cohorts of ready-to-use atrial models to run electrophysiological simulations. The authors describe only how to build the SSM model. The SSM model described therein is not used to fit to subjects (patients) and no practical application is described either.

Even more complicated is the functional twinning phase, where the spatio-temporal myocardial depolarisation is retrieved by clinical data possibly affected by noise and uncertainty. In particular, late gadolinium enhancement distribution and/or electroanatomical maps can be used to infer cardiac tissue characteristics and estimate conduction velocity.

What is missing is a highly automated process that ingests the segmentations as provided for example by CemrgApp and the functional information as provided for example by openEP to build a simulation-ready digital twin model.

A highly automated process to standardize the generation of a subject's atria digital twin from clinical data is therefore desirable.

Zheng et al., "An automated pipeline for generating fiber orientation and region annotation of patient-specific atrial model", Current Directions in Biomedical Engineering, 2021 describe a highly automated process for annotating fibers and anatomical regions in the atria, based on a Laplace-Dirichlet-rule-based-method (LDRBM).

Azzolin et al., "Automated framework for the augmentation of missing anatomical structures and generation of personalized atrial models from clinical data", Computing in Cardiology Conference, 2021 describe a process for generating a personalized atrial model based on electroanantomical mapping data or MRI imaging data, to which the SSM model of Nagel et al. is applied to infer the shape of missing structures by fitting the SSM to the clinically acquired geometries. The model described therein further comprises automated region annotation and fiber generation by applying fiber orientation calculations based on the LDRBM method used by Zheng et al. to deliver ready-to-use models.

A disadvantage of the models described by Zhen et al. (2021) and Azzolin et al. (2021) is the dependency of the output data from the input data. Further, the more specific model described by Azzolin et al. (2021) is limited to the left atrium, while no information from the right atrium are used in the fitting process.

An even further specified model allowing to generate more precise and robust personalized computational models with the highest possible independence from the input data is desired, providing enhances usability and reproducibility and requiring minimal user interaction.

## OBJECT OF THE INVENTION

It was the object of the present invention to provide an improved method for generating a computational atrial model, which overcomes the disadvantages of the prior art. It was a particular object of the invention to provide an improved method for generating a computational atrial model, which provides more freedom with respect to the input data used, e.g. by allowing to use an atrial surface obtained from electroanatomical mapping systems or derived from tomographic imaging segmentation (e.g. MRI or CT). It was a further object of the invention to provide an improved method for generating a computational atrial model, which is further automated, requires a reduced degree of human interaction and/or provides more reliable and reproducible results. Finally, an object of the invention aimed at providing an improved method for generating a computational atrial model, with the highest possible degree of automatization, a minimum of user-interaction, a minimum degree of uncertainty and the highest possible reproducibility, which can be provided in a ready-to-use process based on a variety of available, preferably non-invasive, subject's input data derived from clinical data and which is characterized by the maximum degree of personalization.

These objects of the invention have been solved with the improved method for generating a computational atrial model, developed by the inventors of the present invention and described herein in detail.

## SUMMARY OF THE INVENTION

The present invention is described in more detail below and, in particular, includes the following aspects:

[1] A method for generating a personalized computational model of a subject's heart, comprising the steps:

I-A providing clinical input data from a subject's heart (to be digitally modelled) to provide an output model geometry of the subject's heart;

I-B applying a pre-processing step of automatically removing self-intersections and degenerate elements from the output model geometry to provide a partly modified image having a smoothened geometry (improved model image);

I-C if the input data provide a closed surface, carrying out a step of automatically identifying and opening anatomical openings to provide a model image comprising opened anatomical openings;

II-A labelling of the openings in the model geometry deriving from step I-B or I-C;

III resampling of the model deriving from the previous step (II) for providing an improved quality model,

IV generating fiber orientation in respective anatomical regions of the model deriving from step III,

V carrying out electrophysiological personalization of the structural and anatomical model deriving from the previous steps I to IV, by

V-A applying conduction velocity (CV) modelling via conduction velocity estimation methods, and/or

V-B applying fibrosis modelling (AF-induced remodelling) via image intensity ratio (IIR) or low voltage areas (LVA).

[2] The method according to [1], wherein following step II-A the method comprises the steps

II-B providing a statistical shape model (SSM) of the subject's heart, and

II-C rigidly aligning the SSM with the model image using the labelled openings deriving from step II-A, and

II-D optionally carrying out a further non-rigid fitting procedure.

[3] The method according to [1] or [2], wherein the clinical input data provided in step **I-A** are

a) electroanatomical data or electrophysiological information of the subject's heart, or
b) clinical imaging data of the subject's heart.

[4] The method according to [3], wherein

a) electroanatomical data or electrophysiological information are selected from electroanatomical mapping (EAM) data and electrocardiogram (ECG) data, and/or
b) imaging data are selected from magnetic resonance (MR) data and computer tomography (CT) data.

[5] The method according to one of [1] to [4], wherein the self-intersections and degenerate elements in the input model geometry in step **I-B** are valves and/or veins.

[6] The method according to one of [1] to [5], wherein the anatomical openings in step **I-C** are atrial orifices.

[7] The method according to one of [1] to [6], wherein the pre-processing step **I-B** and the step I-C of opening closed anatomical openings are carried out in varying order or in a combined step (depending on the clinical input data provided in step I-A).

[8] The method according to one of [1] to [7], wherein in step **II-A** the labelling of openings is carried out by labelling both, the right and the left atrium.

[9] The method according to [8], wherein not more than one point per atrium is manually labelled.

[10] The method according to one of [1] to [9], wherein the identification and opening of valve, veins and orifices is carried out manually or automatically.

[11] The method according to one of [1] to [10], wherein in step **I-C** and/or step **II-A** the atrial appendage apex is selected as the one reference point per atrium.

[12] The method according to one of [2] to [11], wherein the SSM is a mono- or bi-atrial statistical shape model, preferably a bi-atrial statistical shape model.

[13] The method according to one of [2] to [12], wherein in step **II-B** a labelling of openings in the SSM is carried out.

[14] The method according to one of [2] to [13], wherein comprising:

(i) the alignment step **II-C** with a rigid alignment between the labelled model image deriving from step II-A with the labelled SSM via the atrial orifice's centers of mass, and/or

(ii) a step of automatic landmark generation in the aligned model deriving from step II-C (i), and

(iii) **II-D** applying a non-rigid shape model fitting algorithm to provide a bi-atrial model.

[15] The method according to [14], wherein in step **II-C (ii)** landmarks are applied by using geodesic paths connecting the marked orifices.

[16] The method according to [14] or [15], wherein > 30 landmarks are applied, preferably > 35 landmarks are applied, more preferably 36 landmarks are applied.

[17] The method according to one of [14] to [16], wherein in step **II-D** the non-rigid shape model fitting is performed by a combination of Iterative Closest Points (ICP) and Gaussian process (GP) regression.

[18] The method according to one of [2] to [17], wherein following step **II-C** or **II-D** the method comprises the step **II-E** of co-registration of multi-modal data by merging multiple data from the subject's clinical input data from step **I-A** to be fitted with the aligned model deriving from step II-C.

[19] The method according to one of [1] to [18], wherein in step **IV** the fiber orientation is generated on a volumetric or a bilayer model, independently from the input data of step I-A, by free selection (of the user).

[20] The method according to one of [1] to [19], wherein in step **V-A** the conduction velocity estimation methods are selected from the group comprising

- a 1st method of discretely applying different CV depending on the IIR value,
- a 2nd method of applying a CV following a regression model relating CV to IIR value,
- a 3rd method of calculating the CV by fitting radial basis functions, and
- a 4th method of iteratively tuning each element conductivity to minimize the root mean squared error (RMSE) between the simulated local activation time (LAT) and the recorded clinical LAT of the patient,

and combinations thereof, among which the 1st, the 2nd and the 4th method are preferred, and among which the 4th method is most preferred.

[21] A data processing device or system comprising means for carrying out the method according to [1] to [20].

[22] The data processing device or system according to [21], wherein the means for carrying out the method according to [1] to [20] is a processor configured to perform the steps I to V as defined in [1] to [20].

[23] A computer program comprising instructions which, when the program is executed by a computer, cause the computer to carry out the steps I to V of the method according to [1] to [20].

[24] A computer-readable data carrier medium comprising instructions which, when executed by a computer, cause the computer to carry out the steps I to V of the method as defined in [1] to [20].

[25] A computer-readable data carrier having stored thereon the computer program according to [23].

[26] The use of the personalized computational heart model deriving from the method as defined in [1] to [20] for carrying out in-silico experiments on a subject's cardiac digital twin.

[27] The use according to [26] for evaluating and/or predicting cardiovascular treatment options and/or personalized therapy options.

[28] The use according to [27], wherein the evaluation of cardiovascular treatment and/or therapy options comprises ablation simulation, evaluation of a (cardiac) arrhythmia, of atrial fibrillation (AF), the assessment of arrhythmia vulnerability, and the assessment of initiation, maintenance and progression of AF.

## DETAILED DESCRIPTION OF THE INVENTION

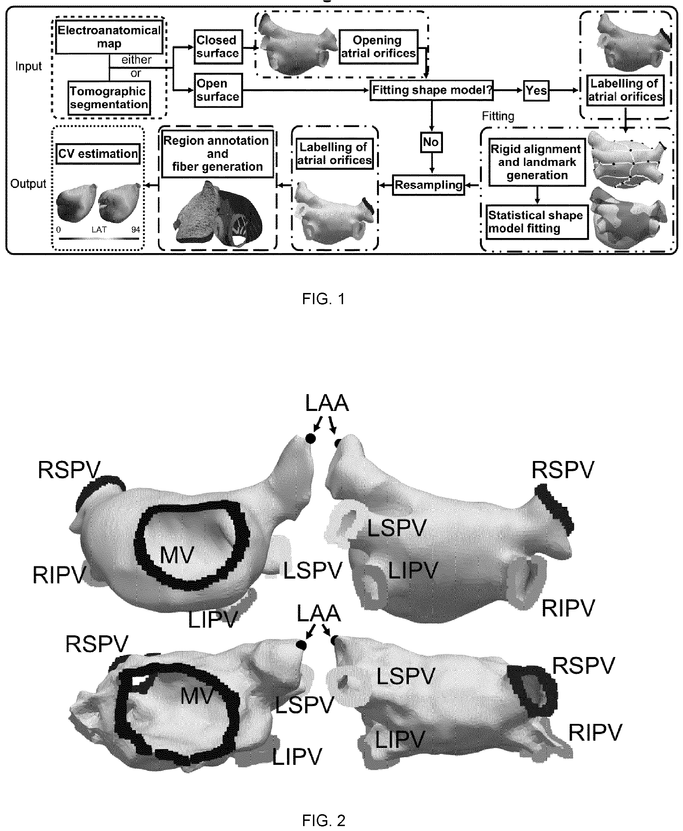

The inventors of the present invention surprisingly found that with the modified and improved method described herein it became possible to provide a new method to standardize the generation of a subject's atrial digital twin from clinical (input) data. The method of the invention provides a new and improved patient-specific augmented atria generation pipeline (herein sometimes also designated "AugmentA") as a highly automated framework which, starting from (conventional) clinical geometrical data (input data), provides ready-to-use atrial personalized computational models. The method of the invention is briefly illustrated by showing the automated modelling pipeline for generating detailed personalized computational models of a human atria in Figure 1.

In summary, the method comprises firstly a pre-processing step applied to the subject's clinical input data (input geometry). Secondly, the atrial orifices are identified and labelled using only very few manually selected reference points, e.g. in the best case one manually selected reference point per atrium is sufficient. If the input data provide a surface with closed orifices, the method comprises a further step of automatically opening of the atrial orifices. Optionally, the user can choose to apply a statistical shape model (SSM) to the input geometry in case augmentation of possibly missing anatomical structures is desired or to resolve segmentation uncertainties. If (applying) fitting a statistical shape model (SSM) to the input geometry is chosen, this is first rigidly aligned with the given mean shape, followed by applying a non-rigid fitting procedure using automatically computed landmarks. Thirdly, the process comprises a step of automatically generating and integrating anatomical structure annotation and fiber orientation. Fourthly, further personalization is carried out by applying local conduction velocity modelling and/or by applying fibrosis modelling to further apply personalized atrial electrophysiology to the digital model. This allows to ensure the closest replica of the patient's depolarization wave propagation

Therewith, the new method provides a single pipeline to standardize the generation of a subject's atrial digital twin from clinical data, which can be applied to a variety of clinical input data (clinical geometrical data) and therewith provides a certain independence from the clinical input data available. The new method is highly automated and requires only a minimum of user-interaction, guaranteeing a high degree of reproducibility and reliability. Due to the additional personalization step the new method allows to provide an improved digital model with the, so far, highest degree of personalization in very short time. Therewith, the new method is able to provide improved ready-to-use atrial personalized computational models.

The method has been tested on a cohort of 29 (human) patients on both segmented magnetic resonance images (MRI) and electroanatomical maps of the left atrium. Moreover, the method was applied to a bi-atrial volumetric mesh derived from MRI. The inventors found that the new and improved method is able to robustly integrate fiber orientation and anatomical region annotations in very short time and with a very low error deviation between in-silico and clinical LAT maps. Therewith, the new method (AugmentA) offers a highly automated and comprehensive pipeline delivering atrial digital twins from clinical data in procedural time making it particularly suitable for real-time application in the clinical workflow.

The new method of the invention exceptionally links together multiple algorithms to automatically generate anatomical and functional atrial digital twins from multiple clinical data. The harmonious communication between all calculation processes allows to build patient-specific models with a minimum of user-interaction. As the processing time of the whole pipeline is well below 1 minute, the method is feasible as in-situ application. The new method offers a fast, easy-to-use, reliable and reproducible platform to generate highly personalized models in normal ablation procedural time.

On particular advantage of the new method is the already mentioned independence from the available input data as with the new method it is possible to provide ready-to-use atrial models from either electroanatomical maps or imaging data (e.g. MRI or CT), while conventional methods are often limited in this respect (e.g. CemrgApp or OpenEP). Another advantage of the new method is the flexibility in possibly applying a SSM fitting, which allows to establish correspondence between a SSM and the clinical geometry at hand. Since the best fitted SSM instance is a deformed version of the mean shape, it always features the same number of nodes and vertex IDs. Therefore, the electrophysiological data mapped from one patient's electroanatomical map to the resulting best fit of the SSM can be easily transferred to the respective SSM instance derived from imaging data. An atrial statistical shape model including variable number of pulmonary veins can be built and easily included in the pipeline, providing additional options for improving the model.

Definitions:

A "subject" according to the invention includes, in principle, humans and animals, e.g. any mammal such as rodents and primates, and in a preferred aspect the subject is human. The term "subject" as used herein can interchangeably be used with the term "patient", in particular in the case of the subjects being humans.
The method described herein involve computers, computer networks or other programmable apparatus, whereby at least one feature is realised by means of a program and is therefore a "computer-implemented method".
The term "computational model" used herein means, in particular, a computer-generated model or (three-dimensional) image of the subject's heart, which is to be modelled with the method of the invention. Therein, "computational" can also be replaced by the term "digital" or "virtual" and the term "model" can also be replaced by the term atrial/cardiac "twin" or "replica". The respective terms can be used interchangeably and accordingly the terms "computational model", "cardiac digital twin" (CDT), "digital atrial model" or "virtual replica" can be used herein interchangeably.

The new Method of the Invention

The new method of the invention comprises the following step:

**Step I-A - Clinical Input Data**

The method firstly requires to provide clinical input data from a subject's heart, which shall be digitally modelled, i.e. from which the digital twin shall be generated. The input can be an atrial surface obtained from conventional and broadly available methods, including on the one hand electroanatomical data or electrophysiological information of the subject's heart, e.g. data derived from electroanatomical mapping systems and on the other hand clinical imaging data of the subject's heart, e.g. from tomographic imaging segmentation (e.g. magnetic resonance (MR) or computer tomography (CT)). Therein, electroanatomical data or electrophysiological information are preferably selected from electroanatomical mapping (EAM) data and electrocardiogram (ECG) data, while imaging data are preferably selected from MR data and CT data. The inventors of the present invention developed a new modelling system, which allows a certain independency of the available input data, as the new method advantageously runs with different kinds of clinical input data.
Providing the clinical input data can take place e.g. by loading the data from a data storage device or by otherwise transferring the data by conventional data transfer methods into the device or computer to run the program for carrying out the new method.

**Step I-B - Mesh pre-processing and removing valves and veins**

Geometries directly derived from clinical data usually do not have a sufficient mesh quality for electrophysiological monodomain simulations. Therefore, regardless of the input, the method of the invention applies a pre-processing step of automatically removing self-intersections and degenerate elements from the model geometry to provide a partly modified image having a smoothened geometry (improved model image). Running such a mesh pre-processing step comprises removing self-intersections and degenerate elements, while regions of the surface without defects are left unmodified. The mesh pre-processing step can for example be carried out using the pymeshfix Python module (https://pypi.org/proiect/pymeshfix/), a MeshFix software which takes as input a polygon mesh and produces a copy of the input where all the occurrences of a specific set of "defects" are corrected. MeshFix has been designed to correct typical flaws present in raw digitized mesh models. The input is assumed to represent a single closed solid object, thus the output will be a single watertight triangle mesh bounding a polyhedron. The self-intersections and degenerate elements in the input model geometry in step I-B are normally and mainly valves and/or veins. The veins are identified as the regions with highest surface curvature, due to their anatomical cylindrical configuration. Briefly, the surface curvature at each node can be calculated as 1/R, where R is the radius of the fitted osculating sphere, as described by L. Azzolin, G. Luongo, S. Rocher, J. Saiz, O. Doessel, and A. Loewe, "Influence of gradient and smoothness of atrial wall thickness on initiation and maintenance of atrial fibrillation", Computing in Cardiology Conference (CinC), 2020. Therein, the parameter R is suitably set, e.g. to 3 cm, in both left (LA) and right atrium (RA). Areas with a surface curvature higher than a predetermined value, e.g. higher than 1.1 times the surface curvature median value, are labelled as high curvature.

**Step I-C - Anatomical Opening Annotation / Mitral Valve Opening**

If the input data provide a closed surface, carrying out a step of automatically identifying and opening anatomical openings to provide a model image comprising opened anatomical openings is applied. Mainly, the anatomical openings in step **I-C** are atrial orifices, including the mitral valve (MV).

For the case of using input data providing a closed surface with no valve openings an automated algorithm is applied to estimate the location of the mitral valve (MV) opening. The method differs depending on whether an electroanatomical map or an MRI segmentation is supplied.

If the input closed surface comes from an electroanatomical map, a method based on peak-to-peak bipolar voltage calculation from all EGM signals of the electroanatomical map in the window of the QRS complex can be used, e.g. by using the so-called ECGdeli toolbox (https://github.com/KIT-IBT/ECGdeli), a Matlab toolbox for filtering and processing single or multilead ECGs. This ECGdeli toolbox filtering functionalities include baseline wander removal techniques, filtering (highpass, lowpass and Notch filter) and isoline correction. The program automatically performs a waveform delineation and can add the timestamps of the onset, peak and offset of the P wave, the QRS complex and the T wave to an FPT table (fiducial point table) for each lead separately or synchronized over all available channels. Areas of high voltage are defined, e.g. values above the 75% voltage percentile, and considered as the MV due to the ventricular far field causing high voltages in this area. To ensure that only the MV is identified and not atrial wall areas, points are only considered if they have high voltage during QRS and a predeterimed low bipolar voltage (e.g. <0.5 mV) outside of the QRS complex.

Since for the closed atrial surface derived from MRI, no electrogram signals are available which can be used to identify the MV, the existing mean shape of the LA where the MV was marked can be used instead. The mean shape is then rigidly aligned and correspondence is established to each new patient geometry. Afterwards, the area marked as MV in the mean shape can then be identified in the new geometry.

The opening of the MV is carried out using an algorithm which is different depending on whether the input geometry comes from tomographic imaging or from electroanatomical mapping.

In the case of a mesh derived from imaging data, the center of mass of the region marked as MV is computed after the co-registration presented in the section above and all the elements with a predetermined distance, e.g. less than 2 cm from the center of mass, are removed to create the opening. Afterwards, the user has to manually select the atrial appendage apex. The modelling process continues with the clipping of the pulmonary veins by removing the elements that belong to high curvature regions and are not including the manually marked left atrial appendage apex.

In the case of an electroanatomical map, all the elements enclosed in a sphere around the center of mass of the region marked as MV and with a diameter computed as the largest distance between all the points labelled as MV are removed. A maximum diameter is set (e.g. 4 cm) as upper bound to be consistent with the dimension of the MV opening from the imaging data. Occasionally, a geometrical artifact caused by the transseptal puncture can be present in the electroanatomical maps. In such cases, the reference point is chosen at the tip of the transseptal puncture instead of the left atrial appendage apex. Later, the veins are identified as the areas with both high curvature and low bipolar voltage (e.g. lower than 0.5 mV), since the left atrial appendage mostly presents high voltage and veins mostly present low voltage. Each vein's ring is generated by clipping all the elements intersecting a sphere centered at the point with maximum curvature and with variable radius chosen as the distance between the highest curvature point and the closest one with a predetermined bipolar voltage (e.g. higher than 0.5 mV). The high curvature region manually marked as geometrical artifact due to the transseptal puncture is left out in the clipping procedure. The remaining high curvature and high voltage regions are annotated as possible left atrial appendage and the vertex with highest curvature as the apex. Following the clipping step, the resulting processed geometry with the final openings and the automatically identified apex of the appendage is visualized with a suitable program, e.g. using PyVista (https://docs.pyvista.org/), a 3D plotting and mesh analysis through a streamlined interface for the Visualization Toolkit (VTK). At this point, the user can decide to manually select a different vertex which is going to be used as reference point in the following labelling step **II-A**.

In the method of the invention, the pre-processing step **I-B** and the step **I-C** of opening closed anatomical openings can be carried out in varying order or in a combined step, depending on the clinical input data provided in step I-A.

**Step II-A** - **Automatic Labelling of Openings**

In step II-A of the method of the invention automatic labelling of the openings in the model geometry deriving from step **I-B** or **I-C** is carried out. Preferably, in step **II-A** the labelling of openings is carried out by labelling both, the right and the left atrium. More preferably, therein not more than one point per atrium is manually labelled. Therein, the identification and opening of valve, veins and/or orifices can be carried out manually or automatically or in a combination of both.

The openings, in particular atrial orifices (pulmonary veins, MV in the LA and inferior & superior vena cava, tricuspid valve, coronary sinus in the RA) can be identified and labelled automatically using for example a clustering algorithm as presented in T. Zheng, L. Azzolin, J. Sanchez, O. Dossel, and A. Loewe, "An automated pipeline for generating fiber orientation and region annotation of patient-specific atrial model," Current Directions in Biomedical Engineering, Vol. 1, No. 1, pages 409-412, 2021 (discussed as prior art above). Then, the only landmarks required are the apex points of the left and right atrial appendages. Briefly, the atrial orifices can be detected as the boundary edges. For the LA, the MV is determined as the largest connected boundary and the rest of the rings are clustered twice to distinguish between left and right as well as inferior and superior pulmonary veins using k-means clustering. The pulmonary veins belonging to the cluster closer to the left atrial appendage (LAA) are labelled as left pulmonary veins (LPV). The remaining cluster is marked as right pulmonary veins (RPV). The LPV are further separated into superior and inferior. The left inferior pulmonary vein is detected as the cluster closest to the LAA. The right inferior pulmonary vein is set as the one belonging to the same side as the left inferior pulmonary vein of the plane passing through the LPV, RPV and the MV centers of mass. For the orifices of the RA, the tricuspid valve (TV) is identified as the largest one. The cluster closest to the apex point of the right atrial appendage (RAA) is labelled as superior vena cava (SVC). The smallest of the remaining two rings is marked as coronary sinus (CS) and the other as inferior vena cava (IVC). Subsequently, a plane passing through the centers of the SVC, IVC and TV is used to identify a band between the IVC and the SVC and to divide the TV into a septal (TV_S) and a lateral wall part (TV_L). These can be used as boundary conditions in the region labelling and fiber generation algorithm as described for step **IV** further below. An example of the resulting annotated geometry in both an MRI segmentation and an electroanatomical map is shown in Figure 2.

In the method according to the invention, in step **I-C** and/or step **II-A** the atrial appendage apex is selected as the one reference point per atrium. Preferably, this atrial appendage apex is labelled manually.

**Step III - Resampling**

A further step of resampling of the model deriving from the previous step **(II),** including its possible sub-steps **II-A** to **II-E**, for providing an improved quality model can be carried out.

Electrophysiological simulations performed with finite elements methods require a fine spatial resolution. Therefore, a mesh resampling step for providing a final high quality mesh with an improved average edge length, e.g. of 0.4 mm, can be carried out. Therefore, a combined smoothing and up-sampling algorithm can be performed using for example one iteration of Laplacian smoothing and for example the isotropic explicit remeshing filter of the PyMeshLab, a Python library interfacing to MeshLab, which is an open source software to edit and process 3D triangular meshes.

**Step IV - Automated Region Annotation and Fiber Generation Algorithm**

The method of the present invention further comprises a step of generating fiber orientation in respective anatomical regions of the model deriving from step **III**.

Atrial fiber architecture is characterized by the presence of multiple overlapping bundles running along different directions, differently from the ventricular fibers architecture where myofibers are aligned along regular patterns. Rule-based methods are widely used strategies to generate myocardial fiber orientation in computational cardiac models. In principle, any suitable myocardial fiber orientation program can be applied here. A particular class of algorithms is known as Laplace-Dirichlet-Rule-Based methods (LDRBM) since they rely on the solution of Laplace problems with Dirichlet boundary conditions. In the method of the present invention, preferably a fully automated method to annotate the different atrial regions and for generating fiber orientation based on LDRBM is implemented. More preferably, LDRBM as described in R. Piersanti, P. C. Africa, M. Fedele, C. Vergara, L. Dedè, A. F. Corno, and A. Quarteroni, "Modeling cardiac muscle fibers in ventricular and atrial electrophysiology simulations," Computer Methods in Applied Mechanics and Engineering, Vol. 373, page 113468, 2021 and in T. Zheng, L. Azzolin, J. Sanchez, O. Dossel, and A. Loewe, "An automated pipeline for generating fiber orientation and region annotation of patient-specific atrial model," Current Directions in Biomedical Engineering, Vol. 1, No. 1, pages 409-412, 2021 are used. Then, six Laplace problems for the LA and six for the RA are formulated:

$$\Delta\psi = \frac{\partial^2\psi}{\partial x^2} + \frac{\partial^2\psi}{\partial y^2} + \frac{\partial^2\psi}{\partial z^2} = 0$$

with proper Dirichlet boundary conditions $\psi_a$ and $\psi_b$ on the respective boundaries $\Gamma_a$ and $\Gamma_b$. These partial differential equations were then solved using the open electrophysiology simulator openCARP. The domain of the Laplace problems is the atrial mesh. The boundary condition domains were obtained using the method described above in connection with step **II-A**. With this method, advantageously, the number of required manually selected seed points can be reduced from four to two (apex of LAA and RAA), thus decreasing user interaction and effort and enhancing reproducibility. The two additional landmarks needed in the previously described method of *Zheng et al. (2021)* on the LAA basis were automatically identified by solving two additional Laplace equations $\psi_{ab2}$ with $\psi_a = 0$ in the RPV and $\psi_b = 1$ at the LAA apex and $\psi_{r2}$ with $\psi_a = 0$ at both LPV and RPV and $\psi_b = 1$ at the MV ring as Dirichlet boundary conditions. In addition, a supplementary Laplace system $\psi_{V2}$ with $\psi_a = 1$ in the IVC and $\psi_b = 0$ at the RAA apex is solved in the RA to improve the identification of the RAA compared to *Zheng et al. (2021).* The complete list of boundary conditions used can be found in Tab.1 in the Example part. Therein, the bundle selection was automatically performed and the bundles' dimension was adjusted for each patient using a region growing method. Following the bundle selection, an orthonormal local coordinate system was built at each element of the atrial domain by performing a Gram-Schmidt orthogonalization. In the case that only an endocardial surface mesh is provided as input, the transversal direction is computed as the normal vector to the atrial surface at each point and the option of generating a bilayer model with different endo- and epicardial fiber arrangement can be included in the process.

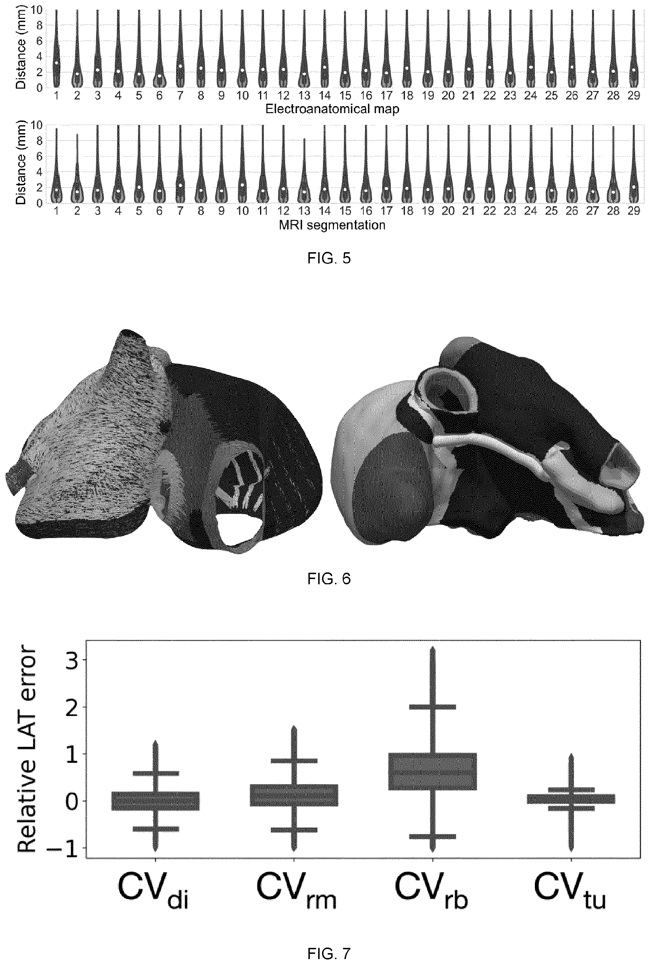

Figure 6 shows an Example of a bi-atrial bilayer model generated with the method of the invention with annotated regions and calculated fiber orientation.

It is a particular advantage of the new method of the present invention, that step **IV** with the fiber orientation can be generated on a volumetric or a bilayer model, independently from the input data of step **I-A**, by free selection of the user.

**Step V - Electrophysiological Personalization**

The method of the present invention is further characterized by a high degree of personalization of the resulting computational model, due to comprising a step of carrying out electrophysiological personalization of the structural and anatomical model deriving from the previous steps **I** to **IV**. This electrophysiological personalization step can bee carried out by applying **(V-A)** conduction velocity (CV) modelling via conduction velocity estimation methods, or **(V-B)** by applying fibrosis modelling (AF-induced remodelling) via image intensity ratio (IIR) or low voltage areas (LVA), or by a combination thereof. These steps V-A and V-B are described in more detail below.

**Step V-A - Conduction Velocity Modelling**

In one aspect, electrophysiological personalization can comprise applying conduction velocity (CV) modelling via known conduction velocity estimation methods. For example, in the method according to the invention, in step **V-A** the conduction velocity estimation methods can be selected from the group comprising

- a 1st method of discretely applying different CV depending on the IIR value,

- a 2nd method of applying a CV following a regression model relating CV to IIR value,

- a 3rd method of calculating the CV by fitting radial basis functions, and

- a 4th method of iteratively tuning each element conductivity to minimize the root mean squared error (RMSE) between the simulated local activation time (LAT) and the recorded clinical LAT of the patient,

and combinations thereof. In the preferred embodiments of the invention, the 3rd method mentioned above is less preferred. Among the mentioned conduction velocity estimation methods the 1st, the 2nd and the 4th method are preferred, and the 4th method is most preferred. As mentioned, any combination thereof is also possible.

Preferably, when applying the above mentioned 1st to 4th method, regional anisotropy ratio is fixed to a predetermined ratio, e.g. 3.75:1, in the LA. In the 1st and 2nd method, preferably non-invasive data coming from the LGE-MRI are used (i.e. IIR). The 3rd and 4th method preferably estimate the CV from the clinically recorded local activation time (LAT) map.

Therein, the 1st method comprises discretely applying different CV depending on the IIR value ($CV_{di}$). The monodomain conductivity is tuned to reach predetermined values, such as e.g. a longitudinal CV of 1.0 m/s in the healthy tissue (IR<1.2), of 0.7 m/s in the interstitial fibrosis areas ($1.2 \leq IIR < 1.32$) and of 0.6 m/s in the dense fibrosis regions ($IIR \geq 1.32$) [M. Beach et al., "Using the universal atrial coordinate system for MRI and electroanatomic data registration in patient-specific left atrial model construction and simulation" Vol. 12738, pages 629-638, 2021 and G. Caixal et al., "Accuracy of left atrial fibrosis detection with cardiac magnetic resonance: correlation of late gadolinium enhancement with endocardial voltage and conduction velocity." EP Europace, 2020]

In the 2nd method a CV following a regression model ($CV_{rm}$) is applied relating CV to IIR value *[G. Caixal et al. (2020)]* :

$$CV = 1\,m/s \cdot exp(0.6731 - 0.9177 * \text{IIR})$$

In the 3rd method, the CV is calculated by fitting radial basis functions ($CV_{rb}$), e.g. as described by C. Nagel, N. Pilia, L. Unger, and O. Dossel, "Performance of different atrial conduction velocity estimation algorithms improves with knowledge about the depolarization patter," in Current Directions in Biomedical Engineering, Vol. 5, No. 1. De Gruyter, 2019, pages 101-104 and M. Mase and F. Ravelli, "Automatic reconstruction of activation and velocity maps from electro-anatomic data by radial basis functions," Conf Proc IEEE Eng Med Biol Soc, Vol. 2010, pages 2608-2611, 2010. Therein, the applied algorithm selects stable catheter positions, finds the local activation times (LAT), considers the wall contact and calculates all CV estimates within the area covered by the catheter.

The 4th (preferred) method comprises iteratively tuning ($CV_{tu}$) each element conductivity to minimize the root mean squared error (RMSE) between the simulated LAT and the recorded clinical LAT. The fiber field used is the one presented in the above description of step IV.

Therein, the clinical LAT map is divided into activation bands from the earliest to the latest activation in steps of 30 ms. However, selecting the location of earliest activation as the very first activated map point or electrogram point can be error-prone. Therefore, preferably the earliest activated point is identified as the center of mass of the region with clinical LAT within the 2.5th percentile. Firstly, the earliest 2.5th percentile LAT points are identified and then their center of mass is calculated and used as earliest activated point in the in-silico experiment. Before proceeding with the iterative fitting of the clinical LAT, the nodes with an earlier activation than the neighbouring vertices are detected and mark as wrong annotations. The LAT annotations in these areas are not used in the fitting process and the conductivity is chosen as the mean of the region boundary.

### Step V-B - Fibrosis Modelling

Alternatively, or in addition, electrophysiological personalization can be achieved by applying fibrosis modelling (AF-induced remodelling), e.g. via image intensity ratio (IIR) or low voltage areas (LVA).

The myocyte membrane dynamics are represented with a variant of the original Courtemanche et al. model *[M. Courtemanche, R. J. Ramirez, and S. Nattel, "Ionic mechanisms underlying human atrial action potential properties: insights from a mathematical model," The American Journal of Physiology, Vol. 225, pages 301-321, 1998]* reflecting AF-induced remodelling. Therein, atrial tissue regions with IIR higher than a predetermined value, e.g. 1.2, are labelled as fibrosis. In the method of the present invention, when applying present step V-B, it turned out to be suitable to set 30% of the elements in the fibrotic regions as almost not conductive (conductivity of $10^{-7}$ S/m) to account for structural remodelling and the presence of scar tissue. This approach modelled the macroscopic passive barrier behaviour caused by the electrical decoupling of the myocytes in the tissue infiltrated by fibrosis, also referred to as 'percolation'. In the other 70%, several ionic conductances were rescaled to consider effects of cytokine-related remodelling (-50% $g_{K1}$, -40% $g_{Na}$ and -50% $g_{CaL}$. The spread of the electrical depolarization in the atrial myocardium can then be simulated by solving the monodomain equation using openCARP and a time step of e.g. 0.02 ms.

### Step II-B - Optional Statistical Shape Model (SSM)

After the labelling of the atrial orifices in step **II-A**, the user can choose whether to proceed with fitting a SSM to the target geometry or directly jump to the resampling step **III** followed by region annotation and fiber generation of step **IV**.

Accordingly, the method of the present invention can, by free selection of the user, following step **II-A**, additionally comprise to provide a statistical shape model (SSM) of the subject's heart (**II-B**). If an SSM is applied, this step (**II-B**) can comprise a labelling of openings (e.g. orifices) in the SSM. Although not mandatorily necessary, this can help to obtain model of even better quality and higher precision.

In one aspect of the method of the invention, wherein the optional SSM is applied, the SSM is a mono- or bi-atrial statistical shape model, preferably a bi-atrial statistical shape model. A possible (suitable) bi-atrial SSM is for example described in the above discussed prior art *Nagel et al. (2021),* the content of which is included herein by reference.

The step of applying an SSM (**II-B**) preferably comprises one or more of the following further steps, including a step **II-C** of rigidly aligning the SSM with the model image using the labelled openings deriving from step **II-A** is applied. To provide even improved SSM instances representing the best (closest) original geometry, it is advantageous to add a further (optional) step **II-D** of carrying out a further non-rigid fitting process and/or a step **II-E** of co-registration of multi-modal data. The steps of the optional SSM are described below in more detail.

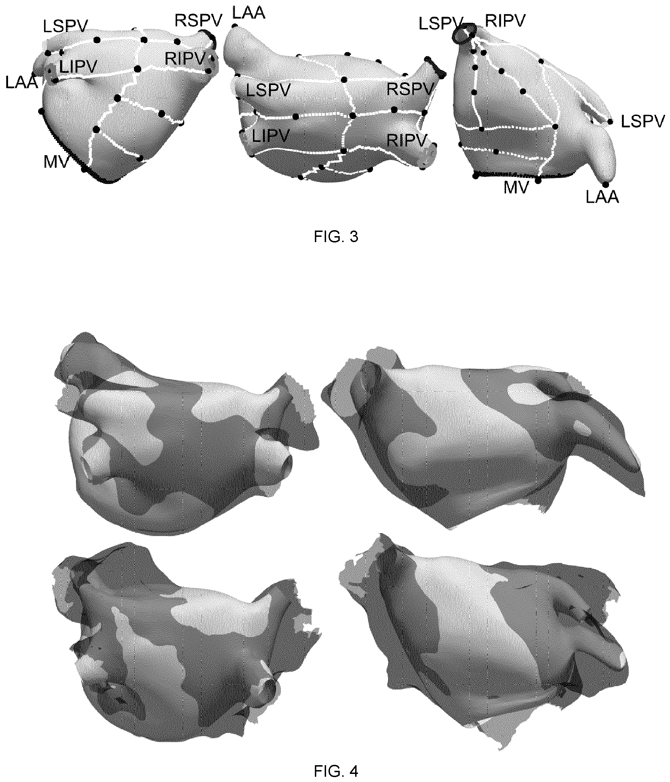

Figure 4 shows an example of non-rigid fitting procedure applied to a SSM instance for each electroanatomical map and MRI segmentation, with Figure 5 showing the surface-to-surface distance between the best fitted SSM and the target electroanatomical map (2.72 ± 2.17 mm) and the surface-to-surface distance in the case of target atrial geometries derived from MRI segmentations (2.13 ± 1.79 mm) across a number of test patients.

### Step II-C - Rigid Alignment and Landmark Generation

In case the user wants to fit a SSM to the target geometry, a rigid alignment in space should be carried out before the fitting procedure since translation and rotation are not represented by eigenmodes of the SSM.

Such alignment step (**II-C**) comprises:

(i) a rigid alignment between the labelled model image deriving from step **II-A** with the labelled SSM via the atrial orifice's centers of mass, and/or

(ii) a step of automatic landmark generation in the aligned model deriving from (i) step **II-C**.

In the rigid alignment step the transformation matrix can be derived by minimizing the weighted sum of squared deviations between the atrial orifices centroids of the two surfaces (**II-C**, (i)). Several characteristic points (landmarks) are then automatically identified in the LA, e.g. by using geodesic paths connecting the previously marked orifices (**II-C**, (ii)). Usually, applying > 30 landmarks can provide suitable results, e.g. as described in the prior art *Azzolin et al. (2021)* discussed above, wherein 35 landmarks are applied. However, the inventors of the present invention surprisingly found that applying more than 35 landmarks (> 35) improves the precision of the resulting target mesh. In particular, applying 36 landmarks turned out to provide good results. The landmarks can, for example, be applied as follows: 10 landmarks in the pulmonary veins, 4 around the MV ring, 4 in the roof, 2 in the septum, 1 in the left lateral wall, 9 in the anterior wall, 5 in the posterior wall, 1 at the LAA apex. This exemplary way of applying 36 landmarks with geodesic paths connecting the atrial orifices labelled in the previous step provides reference points covering most of the atrial surface and allows to localize the most important atrial structures, as shown in Figure 3. However, the number and distribution of the landmarks can be varied in accordance with the knowledge of the skilled person and the desired results and/or depending on the quality of the input geometry.

The accordingly labelled (landmarked) model is then used for the subsequent fitting procedure.

### Step II-D - Non-Rigid Shape Model Fitting

In step **II-D** a non-rigid shape model fitting can be performed by a combination of Iterative Closest Points (ICP) and Gaussian process (GP) regression. Applying a non-rigid shape model fitting algorithm can be used to provide a bi-atrial model.

A preferred embodiment of the inventione aimed at establishing correspondence between a bi-atrial SSM and the clinical geometry at hand. The geometries used therein are usually not included in the process of building the SSM. Non-rigid fitting can be performed by a combination of Iterative Closest Points (ICP) and Gaussian Process (GP) regression. This methodology differs from the typical rigid ICP, which consists of identifying the best rigid transformation between two meshes. Briefly, the main steps of the classical rigid ICP are the following:

- finding candidate correspondences between the moving mesh (i.e. SSM instance) and the target (i.e. clinically derived geometry) by considering the closest point on the target mesh as a candidate;
- solving for the best rigid transform between the moving mesh and the target mesh using Procrustes analysis;
- transforming the moving mesh using this transformation;
- repeating until convergence.

The non-rigid ICP algorithm used in step **II-D** of model fitting int this preferred embodiment of the invention performs the same steps. However, instead of finding a rigid transformation, it solves for a non-rigid one using GP regression. The non-rigid transformation consists of the deformation that is encoded in the SSM eigenmodes. Given a set of points (e.g. belonging to an instance of the SSM), the closest point on the target is attributed as a candidate correspondence to each of the points in the set. The returned sequence of points contains the candidate correspondences to the input points. The first main idea behind ICP is to use the candidate correspondences in a GP regression to find the best model instance explaining the observed deformations even though the correspondences are not perfect. Then a function is defined that, given a sequence of identifiers of model points and their candidate correspondence positions, computes a GP regression based on the resulting deformation field and returns the principal component coefficients of the model instance fitting the candidate deformations best. These coefficients are then used as input to retrieve a bi-atrial geometry that fits the target LA. This way of non-rigid fitting of the used SSM can, for example, be implemented by using ScalismoLab (https://scalismo.org).

**Step II-E** - **Co-Registration of Multi-Modal Data Set**

Following step **II-C** or **II-D** the method of the invention may comprise the step **II-E**, which comprises a co-registration of multi-modal data by merging multiple data from the subject's clinical input data from step **I-A** to be fitted with the aligned model deriving from step **II-C.**

This fitting method establishes correspondence between each target geometry and the SSM. Each data vector (local activation time, voltage, LGE IIR, etc.) is mapped from the original mesh to the fitted SSM using a nearest neighbour algorithm. Since the best fitted SSM instance is a deformed version of the mean shape, it always features the same number of nodes and vertex IDs. Therefore, the electrophysiological data mapped from one patient's electroanatomical map to the resulting best fit of the SSM can be easily transferred to the respective SSM instance derived from imaging data. Since it can be assumed that the MRI segmentation provides a better representation of the patient's real atrial anatomy compared to the electroanatomical mesh, local activation time, uni- and bipolar voltage maps are registered to the SSM instance derived from the MRI fitting.

Means for Carrying out the new Method of the Invention

The present invention further comprises technical means and devices for carrying out the invention in practice.

In particular, the invention includes suitable data processing devices or systems comprising means for carrying out the new method as defined herein.

The invention further includes such data processing devices or systems, wherein the means for carrying out the method of the invention is a processor (or one or more) configured to perform and run the steps I to V (including the sub-steps) as defined herein in detail.

The invention further includes a computer program comprising instructions which, when the program is executed by a computer, causes the computer to carry out the steps I to V (including the sub-steps) as defined herein.

The invention further includes a computer-readable data carrier medium comprising instructions which, when executed by a computer, causes the computer to carry out the steps I to V (including the sub-steps) as defined herein.

The invention further includes a computer-readable data carrier having stored thereon the computer program mentioned above.

The invention further includes medical devices, which may comprise data processing devices and computer programs for carrying out the method of the invention, in particular computer programs leveraging cardiac digital twins.

Use of the new Method of the Invention

The method of the present invention offers new and improved cardiac diagnosis and therapy options, as it can be used to generate patient specific models to be used to test the effect of new drug on arrhythmia termination and to investigate new diagnostics, treatments and/or medical devices. Moreover, it can improve risk stratification and promote patient-specific ablation therapy.

The method of the invention can further improve current approaches focusing on personalized medicine. It can offer new opportunities in combination with non-invasive (e.g. magnetic resonance imaging, computed tomography) as well as invasive (e.g. mapping catheters) clinical mapping systems, by integrating this pipeline in existing workflows to not only provide geometrical and/or electrophysiological information, but deliver an augmented 3D patient-specific model integrating all information derived from clinical data, estimated conduction velocity map visualization to plan targeted and personalized ablation strategies and ready-to-use for computer simulations.

Accordingly, the invention further includes the use of the personalized computational heart model obtainable by performing the method define herein (i.e. deriving from the method of the invention) for carrying out in-silico experiments on a subject's cardiac digital twin.

The use according to the invention is particularly suitable for evaluating and/or predicting cardiovascular treatment options and/or personalized therapy options. Evaluation of cardiovascular treatment and/or therapy options may comprise ablation simulation, evaluation of a (cardiac) arrhythmia, of atrial fibrillation (AF), the assessment of arrhythmia vulnerability, and the assessment of initiation, maintenance, and progression of AF.

**DESCRIPTION OF THE FIGURES**

Fig. 1    Overview of the method of the invention (pipeline) for the generation of personalised computational atrial models from different clinical anatomical data.

Fig. 2    Output of the atrial openings annotation step (mitral valve and roof view of the labelled atrial orifices). Top row: example based on an MRI segmentation. Bottom row: example based on an electroanatomical map. LAA: left atrial appendage apex; MV: mitral valve; LIPV: left inferior pulmonary vein; LSPV: left superior pulmonary vein; RIPV: right inferior pulmonary vein; RSPV: right superior pulmonary vein.

Fig. 3    Posterior, roof and anterior view of the landmarks generation procedure on the left SSM mean instance, including geodesic paths used to identify the landmarks locations in white and respective 36 landmarks in black.

Fig. 4    Top row: Roof and anterior view of the SSM instance (light grey) best fitting to the target MRI segmentation (dark grey). Bottom row: Roof and anterior view of the SSM instance (light grey) best fitting to the target electroanatomical map (dark grey).

Fig. 5    Top row: surface-to-surface distance between the best fitting SSM instance and the respective electroanatomical map. Bottom row: surface-to-surface distance between the best fitting SSM instance and the respective MRI segmentation.

Fig. 6    Posterior view of the fiber orientation computed with with the method of the invention in a bi-atrial bilayer model derived from a SSM and anterior view of the region annotation in a bi-atrial volumetric model in which the wide bachmann bundle is highlighted in white.

Fig. 7    Relative LAT error between simulated and clinical LAT map using the four conduction velocity estimation methods.

## EXAMPLES

### Materials and Methods and Dataset

The highly automated atrial modeling pipeline according to the method of the invention as described herein has been tested on a cohort of 29 persistent AF patients (65+-9 years, 86% male) for which both electroanatomical maps and MRI segmentations of the left atrium (LA) were available. High density electroanatomical left atrial maps (2170+-478 sites) were acquired in sinus rhythm prior to pulmonary vein isolation using a 20-polar mapping catheter (Lasso-Nav or PentaRay-catheter, Biosense Webster Inc., CA, USA) and the CARTO 3 system (Biosense Webster Inc., CA, USA). The post-processing of the LA LGE-MRI (3 Tesla, Somatom Skyra, Siemens Healthcare, Erlangen, Germany)) was performed by an independent expert laboratory (ADAS3D Medical, Barcelona, Spain) blinded to any clinical data. The study was approved by the institutional review board, registered in the German WHO primary registry DRKS (unique identifier: DRKS00014687), and all patients provided written informed consent prior to enrollment. Moreover, a bi-atrial geometry was derived by Subject 3 presented in Krueger et al.. No electroanatomical map was available in this case.

### Results

### Removing Mitral Valve and Pulmonary Veins

The automatic annotation of the MV opening region identified the location of the MV in all 29 patients both in electroanatomical maps and MRI segmentations. Regions with surface curvature higher than 1.1 times the median value identified both veins and appendage in the case of MRI segmentation and the pipeline correctly proceeded with the clipping of the veins regions, though maintaining the appendage. When the input geometry was derived from an electroanatomical map, the combination of high surface curvature and low bipolar voltage resulted in an accurate location of the pulmonary vein regions in the LA. The remaining high curvature area always corresponded to the LAA.

### Atrial orifices labelling

Atrial orifices were automatically identified and correctly labelled from the pipeline in all 29 patients. The accuracy of the labelling was checked by visual inspection. An example of the resulting annotated geometry in both an MRI segmentation and an electroanatomical map is shown in Figure 2. The decision of using only the atrial appendage apexes as reference point for the labelling method turned out to be appropriate to robustly discriminate between valves and veins in both left and right atrium.

**Statistical shape model fitting process**

All 36 landmarks were correctly and automatically identified in the whole patient cohort using the geodesic paths connecting the atrial orifices labelled in the previous step. The reference points covered most of the atrial surface and localized the most important atrial structures, as shown in Figure 3. The non-rigid fitting procedure provided the best fitted SSM instance for each electroanatomical map and MRI segmentation, as presented in Figure 4. Figure 5 shows the surface-to-surface distance between the best fitted SSM and the target electroanatomical map (2.72+-2.17 mm). The surface-to-surface distance in the case of target atrial geometries derived from MRI segmentations was 2.13+-1.79 mm across all patients.

**Atrial region annotation and fiber orientation generation**

Atrial structures were automatically annotated in all 29 LA SSM instances and the pipeline carefully identified each region using the previously labelled atrial orifices and the LAA apex. Finally, bilayer models including realistic fiber orientation were generated. The pipeline was therefore tested on a bi-atrial surface model coming from the mean shape of a SSM. In both LA and RA, regions were correctly annotated. Moreover, fiber orientation was calculated and a bi-atrial bilayer model was generated along with inter-atrial bundles, as presented in Figure 6. In the case of a bi-atrial surface as input, the only two manual reference points needed by the full pipeline are left and right atrial appendage apexes.

Figure 6 shows the results of region annotation and fiber orientation on a volumetric bi-atrial mesh derived from MRI segmentation. The same pipeline was further applied to 100 different volumetric atrial models with homogeneous thickness coming from various instances of a SSM and made publicly available. In *Zheng et al. (2021),* a volumetric bi-atrial mesh with heterogeneous myocardial thickness was used as input and the pipeline proceeded with region annotation and fiber generation.

Table 1 shows the complete list of boundary conditions used:

DIRICHLET BOUNDARY CONDITIONS OF THE TWELVE LAPLACE PROBLEMS.

| Atrium | $\psi$ | $\psi_a$ | $\Gamma_a$ | $\psi_b$ | $\Gamma_b$ |
|---|---|---|---|---|---|
| | $\psi_{tran}$ | 1 | $\Gamma_{epi}$ | 0 | $\Gamma_{endo}$ |
| | $\psi_{ab}$ | 2 | $\Gamma_{rpv}$ | 1 | $\Gamma_{mv}$ |
| | | 0 | $\Gamma_{lpv}$ | -1 | $\Gamma_{ap}$ |
| LA | $\psi_{ab2}$ | 1 | $\Gamma_{ap}$ | 0 | $\Gamma_{rpv}$ |
| | $\psi_v$ | 1 | $\Gamma_{rpv}$ | 0 | $\Gamma_{lpv}$ |
| | $\psi_r$ | 1 | $\Gamma_{mv}$ | 0 | $\Gamma_{lpv} \cup \Gamma_{rpv} \cup \Gamma_{mv}$ |
| | $\psi_{r2}$ | 1 | $\Gamma_{mv}$ | 0 | $\Gamma_{lpv} \cup \Gamma_{lpv}$ |
| | $\psi_{tran}$ | 1 | $\Gamma_{epi}$ | 0 | $\Gamma_{endo}$ |
| | $\psi_{ab}$ | 2 | $\Gamma_{ivc}$ | 1 | $\Gamma_{tv}$ |
| | | 0 | $\Gamma_{svc}$ | -1 | $\Gamma_{ap}$ |
| RA | $\psi_v$ | 1 | $\Gamma_{ivc}$ | 0 | $\Gamma_{svc} \cup \Gamma_{ap}$ |
| | $\psi_{v2}$ | 1 | $\Gamma_{ivc}$ | 0 | $\Gamma_{a\rho}$ |
| | $\psi_r$ | 1 | $\Gamma_{tv}$ | 0 | $\Gamma_{top}$ |
| | $\psi_w$ | 1 | $\Gamma_{tv-s}$ | 0 | $\Gamma_{tv-w}$ |

**Conduction velocity estimation**

The simulated LAT map based on an underlying CV as estimated with the 4th method from the clinical LAT for patient 1. The in-silico LAT maps were computed with all four CV estimation methodologies as defined in context with step **V-A** as defined above in detail (results not shown). The root mean square error (RMSE) between the simulated and the clinical LAT maps was calculated for each of the presented CV estimation method and is presented in Tab. 2:

| CV estimation method | RMSE (ms) |
|---|---|
| $CV_{di}$ | $27.1 \pm 13.6$ |
| $CV_{rm}$ | $36.7 \pm 14.1$ |
| $CV_{rb}$ | $107.5 \pm 44.5$ |
| $CV_{tu}$ | $12.7 \pm 5.8$ |

Moreover, the relative error per patient between the simulated and the clinical LAT map was computed as:

$$\text{Relative LAT error} = \frac{\text{Simulated LAT} - \text{Clinical LAT}}{max\,(\text{Clinical LAT})}$$

and the distributions of the relative LAT error using the various methodologies to estimate CV are shown in Figure 7.

**Processing time**

The full pipeline took 38.4+-5.7 s to complete the opening and labelling of atrial orifices, the fitting process, mesh resampling, the region annotation and fiber generation steps, demonstrating the feasibility of an in-situ application.

**Discussion**

**Opening and labelling atrial orifices**

The pipeline automatically identified and proceeded with the opening of the atrial orifices. In comparison to CemrgApp, in which the user has to manually select the mitral valve and all pulmonary vein regions, the only user-interaction needed by our pipeline is the selection of the atrial appendage apex.

**Statistical shape model fitting**

The SSM fitting step allowed to establish correspondences between each geometry derived from clinical data and the SSM. Regions with higher distance were located mostly close to the appendage. However, the LAA volume is often only partly covered during electroanatomical mapping. The average distance of 2.13 mm between the original MRI segmentation is in the range of one to two voxels omitting segmentation uncertainty. Therefore, the best fitted SSM instance very well represented the original anatomy and augmented the missing anatomical structures (e.g. appendages) compared to the prior art methods.

The SSM generalized well to unseen geometries and accurately represented relevant anatomical features. Moreover, the patient-specific generalization in regions with high inter-individual variability such as the appendages and the PVs could be improved by updating the SSM with more clinical data.

**Region annotation and fiber generation**

The method of the invention provides a highly automated pipeline that accurately generates the fiber direction of the human atria according to histological data. Additionally, it provides a pipeline that annotates the anatomical regions independently from the atrial geometrical variability due to the region growing implementation. It has been demonstrated how the pipeline can generate both volumetric and bilayer 3D models, perform with anatomical variations, and create accurate models of the human atrial anatomy. The pipeline well represents the complex atrial fiber architecture and captures the complex fiber bundles arrangements in both LA and RA. Different endocardial and epicardial fiber orientation can be included to faithfully represent the transmural variability even in a bilayer computational model. Comparing to the existing methods, which require up to 21 manually defined seed points and a longer calculation time, the method of the present invention significantly reduces the calculation time and human manual interaction. The original LDRBM by *Piersanti et al. (2021)* and updated by *Zheng et al.* (2021), both cited above, can be further improved enhancing automation and reproducibility by limiting the manual user interaction to the selection of one reference point per atrium and using an extra set of Laplace solutions. C. H. Roney, R. Bendikas, F. Pashakhanloo, C. Corrado, E. J. Vigmond, E. R. McVeigh, N. A. Trayanova, and S. A. Niederer, "Constructing a human atrial fibre atlas." Annals of biomedical engineering, 2020 presented a methodology to assign the fiber orientation by mapping the fiber field from a human atrial fiber atlas to different patient specific atrial models. Even if the impact of the fiber field on average activation times computed in paced rhythm was relatively small, it had a larger effect on maximum LAT differences. Moreover, arrhythmia dynamics were highly dependent on the fiber field, suggesting that atrial fiber fields should be carefully assigned to patient-specific arrhythmia models. The method of the present invention ensures personalization of both region annotation and specification of fiber orientation to each anatomical structure in the human atria.

**Conduction velocity estimation**

Three state-of-the-art methods to estimate cardiac tissue CV from clinical data have been evaluated systematically and compared to the new iterative fitting to the clinical LAT map algorithm implemented in the method of the present invention. Monodomain conductivity in each element was calculated directly from the CV map using a regression curve representing the relationship between CV and conductivity computed tuning the tissue conductivities to match a specific CV in a tissue slab. This approach can potentially underestimate longitudinal CV if the excitation direction is not completely parallel to the myocyte orientation. This might be part of the reason for the bias towards positive errors. The $CV_{rb}$ method had the highest RMSE due to its sensitivity to sparse data recordings.

**Data availability**

All personalized computational models used in this study including fiber orientation and anatomical labels are available online https://doi.org/10.5281/zenodo.5589289.

**Conclusion**

The method of the present invention as described herein in detail offers a highly automated, comprehensive and reproducible framework to process geometries derived from clinical data and generate atrial anatomical and functional digital twins. The method is able to provide ready-to-use atrial models starting from either electroanatomical maps or imaging data. The method incorporates various region labels and detailed fiber orientation reducing the user-interaction to the selection of one reference point per atrium. The method was moreover tested with surface and volumetric input and it correctly proceeded with atrial structure annotation and fiber generation.

Therewith, the method of the present invention provides a default framework for the generation of atrial digital twin models from clinical data and provides a step forward to improve comparability and reproducibility of atrial models derived from clinical data and to facilitate the evaluation of arrhythmia vulnerability and ablation planning automating the computational model generation steps.

**ABBREVIATIONS**

| AF | atrial fibrillation |
|---|---|
| CS | coronary sinus |
| CT | computer tomography |
| CV | conduction velocity |
| EGM | electrogram |
| GP | Gaussian Process |
| ICP | Iterative Closest Points |
| IIR | image intensity ratio |
| IVC | inferior vena cava |
| LA | left atrium |
| LAA | left atrial appendage |
| LAT | local activation time |
| LDRBM | Laplace-Dirichlet-Rule-Based method(s) |
| LGE | late gadolinium enhancement |
| LIPV | left inferior pulmonary vein(s) |

(continued)

| LPV | left pulmonary vein(s) |
|---|---|
| LSPV | left superior pulmonary vein(s) |
| MR / MRI | magnetic resonance (images) |
| MV | mitral valve |
| RA | right atrium |
| RAA | right atrial appendage |
| RIPV | right inferior pulmonary vein(s) |
| RMSE | root mean squared error |
| RPV | right pulmonary vein(s) |
| RSPV | right superior pulmonary vein(s) |
| SSM | statistical shape model |
| SVC | superior vena cava |
| TV | tricuspid valve |

**Claims**

1. A (computer-implemented) method for generating a personalized computational model of a subject's heart, comprising the steps:

   **I-A** providing clinical input data from a subject's heart (to be digitally modelled) to provide an output model geometry of the subject's heart;
   **I-B** applying a pre-processing step of automatically removing self-intersections and degenerate elements from the output model geometry to provide a partly modified image having a smoothened geometry (improved model image);
   **I-C** if the input data provide a closed surface, carrying out a step of automatically identifying and opening anatomical openings to provide a model image comprising opened anatomical openings, wherein the anatomical openings are preferably atrial orifices;
   **II-A** labelling of the openings in the model geometry deriving from step I-B or I-C;
   **III** resampling of the model deriving from the previous step (II) for providing an improved quality model,
   **IV** generating fiber orientation in respective anatomical regions of the model deriving from step III,
   **V** carrying out electrophysiological personalization of the structural and anatomical model deriving from the previous steps I to IV, by
   **V-A** applying conduction velocity (CV) modelling via conduction velocity estimation methods,
   and/or
   **V-B** applying fibrosis modelling (AF-induced remodelling) via image intensity ratio (IIR) or low voltage areas (LVA).

2. The method according to claim 1, wherein following step II-A the method comprises the steps

   **II-B** providing a statistical shape model (SSM) of the subject's heart, and
   **II-C** rigidly aligning the SSM with the model image using the labelled openings deriving from step **II-A**, and
   **II-D** optionally carrying out a further non-rigid fitting procedure.

3. The method according to one of the preceding claims, wherein the clinical input data provided in step **I-A** are

   a) electroanatomical data or electrophysiological information of the subject's heart, such as data selected from electroanatomical mapping (EAM) data and electrocardiogram (ECG) data
   or
   b) clinical imaging data of the subject's heart, such as data selected from magnetic resonance (MR) data and computer tomography (CT) data.

4. The method according to one of the preceding claims, wherein in step **II-A** the labelling of openings is carried out by labelling both, the right and the left atrium, and wherein preferably not more than one point per atrium is manually labelled.

5. The method according to one of the preceding claims, wherein in step **I-C** and/or step **II-A** the atrial appendage apex is selected as the one reference point per atrium.

6. The method according to one of claims 2 to 5, wherein the SSM is a mono- or bi-atrial statistical shape model, preferably a bi-atrial statistical shape model.

7. The method according to one of claims 2 to 6, comprising:

(i) the alignment step **II-C** with a rigid alignment between the labelled model image deriving from step II-A with the labelled SSM via the atrial orifice's centers of mass, and/or
(ii) a step of automatic landmark generation in the aligned model deriving from step II-C (i), and
(iii) **II-D** applying a non-rigid shape model fitting algorithm to provide a bi-atrial model.

8. The method according to claim 7, wherein in step **II-C (ii)** landmarks are applied by using geodesic paths connecting the marked orifices, wherein preferably > 35 landmarks are applied.

9. The method according to one of claims 2 to 8, wherein in step **II-D** the non-rigid shape model fitting is performed by a combination of Iterative Closest Points (ICP) and Gaussian process (GP) regression,
and/or
wherein following step **II-C** or **II-D** the method comprises the step **II-E** of co-registration of multi-modal data by merging multiple data from the subject's clinical input data from step I-A to be fitted with the aligned model deriving from step II-C.

10. The method according to one of the preceding claims, wherein in step **IV** the fiber orientation is generated on a volumetric or a bilayer model, independently from the input data of step I-A, by free selection (of the user).

11. The method according to one of the preceding claims, wherein in step **V-A** the conduction velocity estimation methods are selected from the group comprising

- a $1^{st}$ method of discretely applying different CV depending on the IIR value,
- a $2^{nd}$ method of applying a CV following a regression model relating CV to IIR value,
- a $3^{rd}$ method of calculating the CV by fitting radial basis functions, and
- a $4^{th}$ method of iteratively tuning each element conductivity to minimize the root mean squared error (RMSE) between the simulated local activation time (LAT) and the recorded clinical LAT of the patient,

and combinations thereof, among which the $1^{st}$, the $2^{nd}$ and the $4^{th}$ method are preferred, and among which the $4^{th}$ method is most preferred.

12. A data processing device or system comprising means for carrying out the method according to claims 1 to 11, wherein preferably the means for carrying out the method according to claims 1 to 11 is a processor configured to perform the steps I to V as defined in the claims 1 to 11.

13. A computer program comprising instructions which, when the program is executed by a computer, cause the computer to carry out the steps I to V of the method according to the claims 1 to 11.

14. A computer-readable data carrier medium comprising instructions which, when executed by a computer, cause the computer to carry out the steps I to V of the method as defined in claims 1 to 11, or a computer-readable data carrier having stored thereon the computer program according to claim 13.

15. The use of the personalized computational heart model deriving from the method as defined in claims 1 to 11 for carrying out in-silico experiments on a subject's cardiac digital twin, such as for evaluating and/or predicting cardiovascular treatment options and/or personalized therapy options, including ablation simulation, evaluation of a (cardiac) arrhythmia, of atrial fibrillation (AF), the assessment of arrhythmia vulnerability, and the assessment of initiation, maintenance and progression of AF.

...

FIG. 1

FIG. 2

FIG. 3

FIG. 4

FIG. 5

FIG. 6

FIG. 7

**EUROPEAN SEARCH REPORT**

Europäisches Patentamt
European Patent Office
Office européen des brevets

Application Number

EP 22 15 5540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/103865 A1 (TRAYANOVA NATALIA A [US] ET AL) 19 April 2018 (2018-04-19) * paragraph [0017] * * paragraph [0035] * * paragraph [0039] * * paragraph [0041] * * paragraph [0043] * * paragraph [0051] * * paragraph [0093] * * figure 12A * | 1-15 | INV. G16H30/40 G06T17/00 G16H50/50 |
| X | WO 2021/207261 A9 (UNIV JOHNS HOPKINS [US]) 11 November 2021 (2021-11-11) * paragraph [0038] - paragraph [0043] * | 1-15 | |
| X | US 2016/058520 A1 (YANG HUANHUAN [US] ET AL) 3 March 2016 (2016-03-03) * paragraph [0018] * * paragraph [0021] - paragraph [0022] * | 1-15 | |
| X | ALEJANDRO LOPEZ-PEREZ ET AL: "Three-dimensional cardiac computational modelling: methods, features and applications", 17 April 2015 (2015-04-17), BIOMEDICAL ENGINEERING ONLINE, BIOMED CENTRAL LTD, LONDON, GB, PAGE(S) 35, XP021221389, ISSN: 1475-925X *Figure 3 Associated passages*; figure 3 | 1-15 | TECHNICAL FIELDS SEARCHED (IPC) G16H G06T |

-/--

The present search report has been drawn up for all claims

| Place of search | Date of completion of the search | Examiner |
|---|---|---|
| Munich | 11 July 2022 | Rivera Farina, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 22 15 5540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHENG TIANBAO ET AL: "An automate pipeline for generating fiber orientation and region annotation in patient specific atrial models", CURRENT DIRECTIONS IN BIOMEDICAL ENGINEERING, vol. 7, no. 2, 1 October 2021 (2021-10-01), pages 136-139, XP55937537, DOI: 10.1515/cdbme-2021-2035 * the whole document * | 1-15 | |
| X | MARTIN W. KRUEGER ET AL: "Patient-specific modeling of atrial fibrosis increases the accuracy of sinus rhythm simulations and may explain maintenance of atrial fibrillation", JOURNAL OF ELECTROCARDIOLOGY., vol. 47, no. 3, 21 November 2013 (2013-11-21), pages 324-328, XP055373745, XX ISSN: 0022-0736, DOI: 10.1016/j.jelectrocard.2013.11.003 * the whole document * | 1-15 | |
| X | AZZOLIN LUCA ET AL: "Automated Framework for the Augmentation of Missing Anatomical Structures and Generation of Personalized Atrial Models from Clinical Data", 2021 COMPUTING IN CARDIOLOGY (CINC), CREATIVE COMMONS, vol. 48, 13 September 2021 (2021-09-13), pages 1-4, XP033999436, DOI: 10.23919/CINC53138.2021.9662846 [retrieved on 2021-12-23] * the whole document * | 1-15 | TECHNICAL FIELDS SEARCHED (IPC) |

The present search report has been drawn up for all claims

| Place of search | Date of completion of the search | Examiner |
|---|---|---|
| Munich | 11 July 2022 | Rivera Farina, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 5540

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2022

| Patent document cited in search report | Publication date | Patent family member(s) | Publication date |
|---|---|---|---|
| US 2018103865 A1 | 19-04-2018 | US 2018103865 A1<br>WO 2016183385 A1 | 19-04-2018<br>17-11-2016 |
| WO 2021207261 A9 | 11-11-2021 | NONE | |
| US 2016058520 A1 | 03-03-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CORRADO et al.** A work flow to build and validate patient specific left atrium electrophysiology models from catheter measurements. *Medical Image Analysis,* 2018
- **PHEIFFER et al.** Estimation of Local Conduction Velocity from Myocardium Activation Time: Application to Cardiac Resynchronization Therapy. *International Conference on Functional Imaging and Modelling of the Heart,* 2017
- **BOYLE et al.** Computationally guided personalized targeted ablation of persistent atrial fibrillation. *Nat. Biomed. Eng.,* 2019
- **STEGMANN et al.** *Europace,* 2018
- **NAGEL et al.** A bi-atrial statistical shape model for large-scale in silico studies of human atria: model development and application to ECG simulations. *Medical Image Analysis,* 2021
- **ZHENG et al.** An automated pipeline for generating fiber orientation and region annotation of patient-specific atrial model. *Current Directions in Biomedical Engineering,* 2021
- **AZZOLIN et al.** Automated framework for the augmentation of missing anatomical structures and generation of personalized atrial models from clinical data. *Computing in Cardiology Conference,* 2021
- **L. AZZOLIN ; G. LUONGO ; S. ROCHER ; J. SAIZ ; O. DOESSEL ; A. LOEWE.** Influence of gradient and smoothness of atrial wall thickness on initiation and maintenance of atrial fibrillation. *Computing in Cardiology Conference (CinC),* 2020
- **T. ZHENG ; L. AZZOLIN ; J. SANCHEZ ; O. DOSSEL ; A. LOEWE.** An automated pipeline for generating fiber orientation and region annotation of patient-specific atrial model,. *Current Directions in Biomedical Engineering,* 2021, vol. 1 (1), 409-412
- **R. PIERSANTI ; P. C. AFRICA ; M. FEDELE ; C. VERGARA ; L. DEDÈ ; A. F. CORNO ; A. QUARTERONI.** Modeling cardiac muscle fibers in ventricular and atrial electrophysiology simulations,. *Computer Methods in Applied Mechanics and Engineering,* 2021, vol. 373, 113468
- **T. ZHENG ; L. AZZOLIN ; J. SANCHEZ ; O. DÖSSEL ; A. LOEWE.** An automated pipeline for generating fiber orientation and region annotation of patient-specific atrial model,. *Current Directions in Biomedical Engineering,* 2021, vol. 1 (1), 409-412
- **M. BEACH et al.** *Using the universal atrial coordinate system for MRI and electroanatomic data registration in patient-specific left atrial model construction and simulation,* 2021, vol. 12738, 629-638
- **G. CAIXAL et al.** Accuracy of left atrial fibrosis detection with cardiac magnetic resonance: correlation of late gadolinium enhancement with endocardial voltage and conduction velocity. *Europace,* 2020
- **C. NAGEL ; N. PILIA ; L. UNGER ; O. DOSSEL.** Performance of different atrial conduction velocity estimation algorithms improves with knowledge about the depolarization patter,. *Current Directions in Biomedical Engineering,* 2019, vol. 5 (1), 101-104
- **M. MASE ; F. RAVELLI.** Automatic reconstruction of activation and velocity maps from electro-anatomic data by radial basis functions,. *Conf Proc IEEE Eng Med Biol Soc,* 2010, vol. 2010, 2608-2611
- **M. COURTEMANCHE ; R. J. RAMIREZ ; S. NATTEL.** Ionic mechanisms underlying human atrial action potential properties: insights from a mathematical model,. *The American Journal of Physiology,* 1998, vol. 225, 301-321
- **C. H. RONEY ; R. BENDIKAS ; F. PASHAKHANLOO ; C. CORRADO ; E. J. VIGMOND ; E. R. MCVEIGH ; N. A. TRAYANOVA ; S. A. NIEDERER.** Constructing a human atrial fibre atlas. *Annals of biomedical engineering,* 2020